Europäisches Patentamt

European Patent Office

Office européen des brevets

(11) Publication number: **0 214 833 B1**

# EUROPEAN PATENT SPECIFICATION

(45) Date of publication of patent specification: **29.01.92**  (51) Int. Cl.⁵: **H04N 5/32**, **A61B 6/00**

(21) Application number: **86306790.6**

(22) Date of filing: **02.09.86**

(54) **High-resolution radiation image detecting apparatus.**

(30) Priority: **02.09.85 JP 194091/85**

(43) Date of publication of application:
**18.03.87 Bulletin  87/12**

(45) Publication of the grant of the patent:
**29.01.92 Bulletin  92/05**

(84) Designated Contracting States:
**DE GB**

(56) References cited:
**FR-A- 2 565 053**
**GB-A- 2 101 444**
**US-A- 4 247 780**

(73) Proprietor: **SHIMADZU CORPORATION**
**378, Ichinofunairi-cho Kawaramachi-dori Nijo Sagaru**
**Nakagyo-ku Kyoto 604(JP)**

(72) Inventor: **Kiri, Motosada**
**14-11, 6-chome Ooe Nishi Shinbayashi-cho**
**Nishikyo-ku Kyoto 610-11(JP)**

(74) Representative: **Marlow, Nicholas Simon et al**
**Reddie & Grose 16, Theobalds Road**
**London WC1X 8PL(GB)**

# Description

The present invention relates to an electromagnetic radiation image detecting apparatus having its radiation image receiving plane composed of a two-dimensional array of unit radiation sensors constituting picture elements, and more particularly to such an apparatus improved in resolving power by making use of picture signals obtained with the radiation image projection displaced by half an arrangement pitch of the unit radiation sensors, namely of the picture elements.

As an example of radiation image detecting apparatus there is known an X-ray image detecting apparatus devised so as to electronically picture an X-ray image projected on an X-ray image receiving plane consisting of a two-dimensional array of unit X-ray sensors, which form the picture elements of the X-ray receiving plane.

In such an electromagnetic radiation image detecting apparatus the resolving power is in general given by the arrangement pitch of the unit radiation sensors constituting the radiation image receiving plane. Since the arrangement pitch is determined mainly by the size of the unit sensors, it may well be said that the resolving power is given by the size of the sensors. Therefore, in order to increase the resolving power it is necessary to constitute the radiation image receiving plane with as small unit sensors as possible. However, there is imposed a limit, as a matter of course, on the size of the unit sensors by a technological or the economic reason.

US-A-4 247 780 discloses a radiation image detecting apparatus comprising a two-dimensional array of radiation sensors arranged at a constant pitch in each dimension, means for displacing the radiation source relative to the array and a memory for retaining the signals for the sensors.

The present invention aims at improving the resolving power of a radiation image detecting apparatus over the limitation made by the size and arrangement pitch of the unit sensors constituting the picture element of the image receiving plane of the apparatus, and makes it an object of the present invention to provide a radiation image detecting apparatus improved in resolving power with the unit sensor size kept unchanged.

Another object of the present invention is to realize such an improved radiation image detecting apparatus with a method of increasing the resolving power by making use of picture signals obtained with a radiation image projection displaced by half the arrangement pitch of the unit sensors constituting the radiation image receiving plane of the apparatus.

A further object of the present invention is to constitute such an improved radiation image detecting apparatus so that the above mentioned radiation image projection displacement may be affected by displacing the radiation source, thereby enabling the apparatus to provide a high-resolution image picture selectively with respect to a specific plane in an objective whose inner structure is under investigation.

According to the present invention there is provided a radiation image detecting apparatus comprising a radiation image receiving plane composed of a two-dimensional array of radiation sensors arranged at a constant pitch in each dimension; a memory for memorizing signals from the radiation sensors; and means for displacing the radiation source relative to the image receiving plane so that a radiation image projected on the radiation image receiving plane is shifted in both dimensions of the array of radiation sensors, said radiation image receiving plane being adapted for receiving an image from an objective irradiated with radiation from a radiation source;

characterized in that the apparatus further comprises computing means for reading out the image signals stored in the memory and superimposing them on the image signals obtained after shifting the radiation source such that the radiation image projected on the radiation image receiving plane (2) is shifted in both dimensions of the array of radiation sensors (2a) by half the arrangement pitch of the radiation sensors in each dimension respectively.

The principle and details of the present invention are described in the following on reference to the accompanying drawings, in which:

Fig. 1 shows block-diagrammatically the constitution of an embodiment of the present invention;

Fig. 2 is a partial frontal view of the radiation image receiving plane employed in the above embodiment;

Fig. 3 shows a representative unit radiation sensor in the above radiation image receiving plane;

Fig. 4 shows diagrammatically the addresses and subaddresses in a memory used in the above embodiment;

Fig. 5 illustrates the resolving power of a conventional radiation image detecting apparatus having its radiation image receiving plane composed of an array of unit radiation sensors; and

Fig. 6 illustrates the improved resolving power of the present invention.

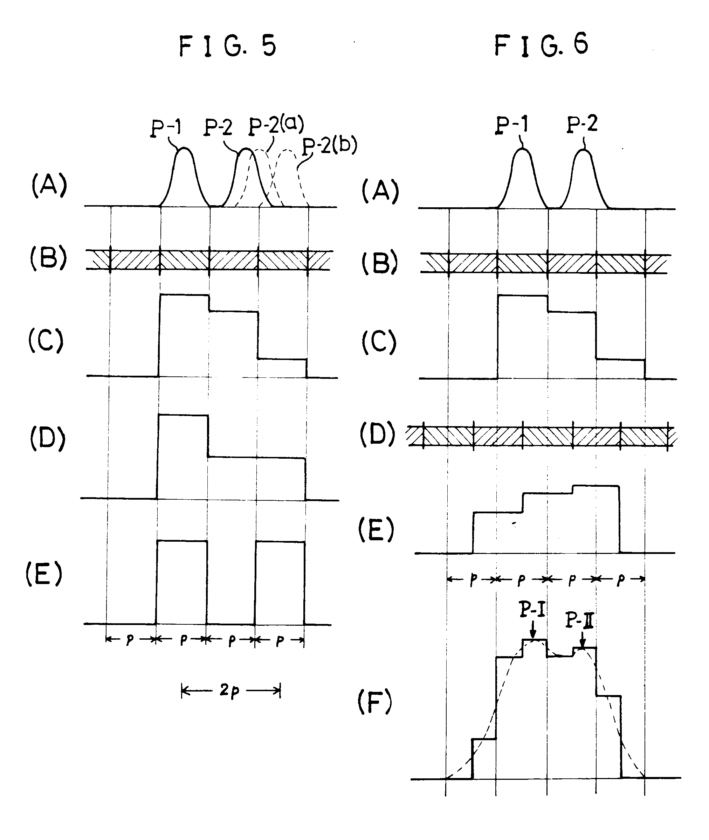

In advance of proceeding with the detailed description of the invention, the principle of the invention is briefed on reference to Figs. 5 and 6, which illustrate the resolving power of a conventional radiation image detecting apparatus and that of the apparatus according to the present invention, respectively. In both Figs. 5 and 6, solid and dotted curves shown in (A) represent intensity distributions of one-dimensional point images P-1 and P-2 (P-2-(a), P-2(b)) projected on radiation image receiving planes shown in (B) (of Figs. 5 and 6) and (D) (of Fig. 6). The image receiving planes consists of one-dimensional array of unit radiation sensors, which are distinguished from adjacent ones with different hatchings showing their respective cross-sections. The arrangement pitch of the unit sensors is p, which is, in these drawings, equal to the width of the sensors because the clearances between the sensors are ignored. The step-like and column-patterns in Figs. 5 and 6 represent the electric output signals from the unit sensors, except for the Fig. 6(F) pattern which is, as is mentioned later, the superposition of the two outputs shown in Figs. 6-(C) and (E).

Referring to Fig. 5 (conventional), the two point images P-1 and P-2 are projected on the image receiving plane about (1 + 1/4)p apart from each other, causing the unit sensors to output image signals as shown in (C). As P-2 goes away from P-1 further rightward through a position P-2(a) (one and half a pitch distant from P-1) to a position P-2-(b) on the right end (just two pitches distant from P-1), the image signals outputted from the unit sensors vary as shown in (D) and (E). According to a conventional apparatus, as is understood from the sensor output variations shown in (C), (D) and (E), the two point images are expected to start being discriminated when the distance between the two exceeds (1 + 1/2)p, and are discriminated completely (refer to (E)) for the first time when P-2 comes to the P-2(b) position a distance 2p apart from P-1.

Also in case of the present invention, whose principle is illustrated in Fig. 6, two point images P-1 and P-2 (Fig. 6(A)) separating from each other by the same distance (1 + 1/4)p as in case of Fig. 5 are first detected with the unit sensors kept as they are (refer to Fig. 6 (B)). The outputs from the sensors show, as a matter of course, the same signal pattern (Fig. 6(C)) as in case of Fig. 5. According to the present invention, however, the outputs thus obtained are temporarily stored in a memory. Secondly, the point images P-1 and P-2 are again detected with the sensors displaced by a distance of (1/2)p (refer to Fig. 6(D)). This (1/2)p-displacement of the sensors causes them to output image signals different, as is shown in Fig. 6(E), from those shown in Fig. 6(C) and stored previously in the memory. The stored signals (Fig. 6-(C)) are read out and superimposed on the signals shown in Fig. 6(E) to obtain a picture signal as shown in Fig. 6(F). According to this picture signal, the two point images P-1 and P-2 are pictured, for instance, on a CRT screen. A dotted curve smoothing this (step-like) picture signal represent an expected real brightness variation on the CRT screen.

As is clearly witnessed, the picture signal has two peaks P-I and P-II enabling the two point image P-1 and P-2 to be discriminated from each other. Although a dip between the two peaks P-I and P-II is still shallow in the present picture signal, the appearance of the separation between the two peaks means that the apparatus according to the present invention is improved in resolving power so that it may begin to discriminate two point images if the separation between them exceeds the sensor arrangement pitch p. The image detecting procedure with the sensors displaced by (1/2) p is of course taken two-dimensionally in practice, though the above explanation of principle is of one-dimension.

The present invention is described in detail with an X-ray image detecting apparatus exemplified as an embodiment of the invention.

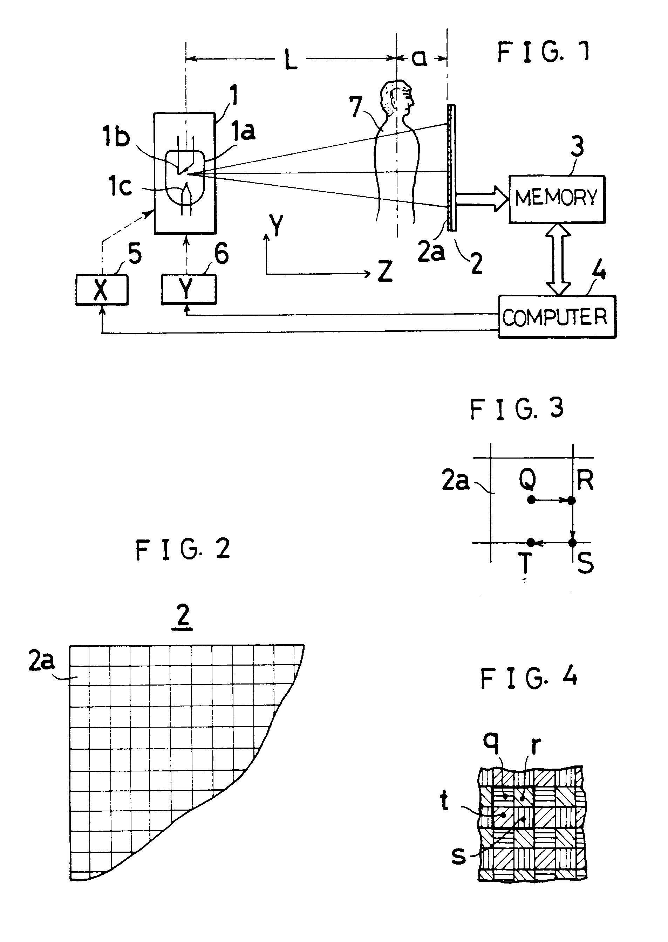

Referring to Fig. 1, which shows block-diagrammatically the constitution of the X-ray image detecting apparatus, X-rays radiated from an X-ray source 1 penetrates an object 7 and projects an inner image of the objective 7 on an X-ray receiving plane 2 consisting of 256 x 256 quadrate unit X-ray sensors 2a constituting the picture elements of the X-ray receiving plane, whose partial frontal view is shown in Fig. 2. The outputs from the unit X-ray sensors 2a are transmitted to a microcomputer 4 through a memory 3. Two X-ray source displacing means 5 and 6 are mechanical systems for displacing the X-ray source 1 respectively in the X-direction and in the Y-direction (the system of coordinates is shown in Fig. 1) along the horizontal and vertical arrangement directions of the unit X-ray sensors 2a. The X-ray source displacing means 5 and 6 are operated by their respective instruction signals given by the microcomputer 4. The X-ray source 1 consists of an X-ray tube 1a, whose anode and cathode are indicated by reference signs 1b and 1c, respectively. The memory 3 is provided with 256 x 256 main addresses corresponding to the 256 x 256 unit X-ray sensors 2a, and each of the main addresses has four subaddresses q, r, s and t as shown diagrammatically in Fig. 4. Thus the total number of the subaddresses amounts to 512 x 512, which is four times the number of the unit sensors. In Fig. 4 the reference signs q, r, s and t are applied only to the subaddresses belonging to one representative main address corresponding to one representative unit X-ray sensor shown in Fig. 3. Reference signs Q, R, S and T given to the representative unit X-ray sensor of Fig. 3 are made clear in the following descriptions.

In the above described constitution of the embodiment, the inner structure of the objective 7 is first projected on the X-ray receiving plane 2 with the X-ray source 1 fixed at a predetermined initial position, and the image signals outputted from the unit X-ray sensors 2a are stored by the memory 3 at the subaddresses q in the main addresses corresponding to the respective sensors 2a. In this case, suppose that one of the X-rays radiated from the source 1 is projected at the central point Q of the representative unit X-ray sensor shown in Fig. 3. Secondly, a similar projection is made with the X-ray source 1 displaced so that the X-ray projected previously at the point Q may be directed to point R. According to such a displacement of the X-ray source 1, all the X-rays projected previously at the points corresponding to Q in the unit sensors other than the representative one are also moved to the points corresponding to R, and this means that the entire X-ray image is displaced horizontally (in the X-direction) by half the arrangement pitch p of the unit sensors if the clearance between adjacent sensors is ignored. The image signals thus obtained, which correspond just to the signals shown in Fig. 6(E), are then stored by the memory 3 at subaddresses r. In order to execute such displaced image detection procedures two-dimensionally, the X-ray image projections are carried out with the X-ray source 1 displaced so as to make said initial X-ray projection direct further to points S and T of Fig. 3. The obtained image signals are stored in the subaddresses s and t corresponding to the X-ray directions shifted to S and T. After all the subaddresses q, r, s and t in the memory 3 have been filled with image signals, the microcomputer 4 reads out the image signals to picture the detected X-ray image on a conventional CRT. In the above description the displacements of the X-ray source 1 are equivalent to the sensor displacement described in conjunction with Fig. 6 illustrating the principle of the present invention. The CRT and related electronics, which are well-known, are deleted in Fig. 1 for the simplification of the drawing.

According to the present invention, the displacement quantity $\xi$ of the X-ray source 1 is determined by a proportional equation $\xi = (1/2) \cdot pL/a$, where L is the distance between the X-ray source and a specific plane in the objective and a is the distance between the specific plane and the X-ray receiving plane (L + a = constant. Refer to Fig. 1). Therefore, it is possible to selectively emphasize the resolving power of the image in a specific plane in the objective by choosing L and a so as to define the specific plane.

The above embodiment can be modified, if the X-ray tube 1a is provided with a plurality of cathodes, by making an X-ray projection point shift through the selection of the suitably positioned cathode.

Further, the present invention can be embodied with the above X-ray image receiving plane 2 replaced with a combination of an X-ray fluorescent plate (or an X-ray image intensifier) and a TV camera. In this case an X-ray image to be detected is projected on the X-ray fluorescent plate (or the X-ray image intensifier), which is not constituted with picture elements, but made of a continuous single body. Therefore, an analog image signal outputted from the TV camera is sampled corresponding to the image signals outputted from the individual unit sensors 2a in case of the embodiment shown in Fig. 1, and then stored in the memory 3. The value of sampling intervals corresponds to the arrangement pitch p of the unit sensors 2a.

## Claims

1. A radiation image detecting apparatus comprising a radiation image receiving plane (2) composed of a two-dimensional array of radiation sensors (2a) arranged at a constant pitch in each dimension; a memory (3) for memorizing signals from the radiation sensors (2a); and means (5,6) for displacing the radiation source (1) relative to the image receiving plane (2) so that a radiation image projected on the radiation image receiving plane (2) is shifted in both dimensions of the array of radiation sensors (2a), said radiation image receiving plane (2) being adapted for receiving an image from an objective (7) irradiated with radiation from a radiation source (1);

   characterized in that the apparatus further comprises computing means (4) for reading out the image signals stored in the memory (3) and superimposing them on the image signals obtained after shifting the radiation source (1) such that the radiation image projected on the radiation image receiving plane (2) is shifted in both dimensions of the array of radiation sensors (2a) by half the arrangement pitch of the radiation sensors in each dimension respectively.

2. A radiation image detecting apparatus according to claim 1 characterized in that it is adapted for detecting an X-ray image.

3. A radiation image detecting apparatus according to claim 1 or 2 in which the means (5,6) for displacing the radiation source (1) relative to the image receiving plane (2) displaces the radiation source horizontally and vertically.

## Revendications

1. Appareil de détection d'image de rayonnement comportant un plan de réception d'image de rayonnement (2) constitué par un réseau bidimensionnel de capteurs de rayonnement (2a) disposé avec un pas constant dans chaque dimension ; une mémoire (3) destinée à mémoriser des signaux provenant des capteurs de rayonnement (2a) ; et des moyens (5, 6) pour déplacer la source de rayonnement (1) par rapport au plan de réception d'image (2) de manière qu'une image de rayonnement projetée sur le plan de réception d'image de rayonnement (2) soit décalée dans deux dimensions du réseau de capteurs de rayonnement (2a), ledit plan de réception d'image de rayonnement (2) étant agencé pour recevoir une image provenant d'un objectif (7) irradié par un rayonnement provenant d'une source de rayonnement (1) ;

caractérisé en ce que l'appareil comporte en outre des moyens de calcul (4) destinés à lire les signaux d'image mémorisés dans la mémoire (3) et à leur superposer les signaux d'image obtenus après décalage de la source de rayonnement (1), de manière que l'image de rayonnement projetée sur le plan de réception d'image de rayonnement (2) soit décalée dans deux dimensions du réseau de capteurs de rayonnement (2a) de la moitié du pas de la disposition des capteurs de rayonnement, respectivement dans chaque dimension.

2. Appareil de détection d'image de rayonnement selon la revendication 1, caractérisé en ce qu'il est agencé pour détecter une image de rayons X.

3. Appareil de détection d'image de rayonnement selon la revendication 1 ou 2, dans lequel les moyens (5,6) de décalage de la source de rayonnement (1) par rapport au plan de réception d'image (2) décalent horizontalement et verticalement la source de rayonnement.

**Patentansprüche**

1. Strahlungsbilderfassungsvorrichtung mit einer Strahlungsbildempfangsebene (2), die aus einer zweidimensionalen Anordnung von Strahlungssensoren (2a) zusammengesetzt ist, welche in jeder Dimension in gleichen Abständen angeordnet sind; einem Speicher (3) zum Speichern der von den Strahlungssensoren (2a) kommenden Signale; und Einrichtungen (5, 6) zum Verlagern der Strahlungsquelle (1) gegenüber der Bildempfangsebene (2), so daß ein auf die Strahlungsbildempfangsebene (2) projiziertes Strahlungsbild in beiden Dimensionen der Anordnung von Strahlungssensoren (2a) verschoben wird, wobei die genannte Strahlungsbildempfangsebene (2) für den Empfang eines Bildes von einem Objekt (7) ausgebildet ist, das mit Strahlung von einer Strahlungsquelle (1) bestrahlt wird;

dadurch gekennzeichnet, daß zu der Vorrichtung außerdem Computereinrichtungen (4) gehören, die dazu dienen, die in dem Speicher (3) gespeicherten Bildsignale auszulesen und sie den nach dem Verschieben der Strahlungsquelle (1) gewonnenen Bildsignalen zu überlagern, so daß das auf die Strahlungsbildempfangsebene (2) projizierte Strahlungsbild in beiden Dimensionen der Anordnung von Strahlungssensoren (2a) in jeder Dimension jeweils um die Hälfte des Anordnungsabstandes der Strahlungssensoren verschoben wird.

2. Strahlungsbilderfassungsvorrichtung nach Anspruch 1, dadurch gekennzeichnet, daß sie für das Erfassen eines Röntgenbildes ausgebildet ist.

3. Strahlungsbilderfassungsvorrichtung nach Anspruch 1 oder 2, dadurch gekennzeichnet, daß die Einrichtungen (5, 6) zum Verlagern der Strahlungsquelle (1) gegenüber der Bildempfangsebene (2) die Strahlungsquelle horizontal und vertikal verschieben.

FIG. 1

L    a

FIG. 3

FIG. 2

FIG. 4

# F I G. 5

(A) P-1 P-2 P-2(a) P-2(b)

(B)

(C)

(D)

(E)

|← p →|← p →|← p →|← p →|

|← 2p →|

# F I G. 6

(A) P-1 P-2

(B)

(C)

(D)

(E)

|← p →|← p →|← p →|← p →|

(F) P-I P-II